# EUROPEAN PATENT APPLICATION

(11) **EP 4 744 718 A1**
(43) Date of publication of application: **20.05.2026**
(21) Application number: 24383253.2
(22) Date of filing: 19.11.2024
(51) Int. Cl.: A61N 1/05

(54) **ELECTRODE MODULE**

(71) Applicant: INBRAIN Neuroelectronics SL, 08028 Barcelona (ES)
(72) Inventor: Bakker, Jurriaan, 5591 TG Heeze (NL)
(74) Representative: DTS Patent- und Rechtsanwälte PartmbB

(57) **Abstract**

The present invention relates to an electrode module 100 for stimulation of the central and/or peripheral nervous system and/or recording of one or more signals of the central and/or peripheral nervous system. The electrode module comprises an electrode module per-product 120, in particular a stimulator lead, including a substrate 110 and at least one electrode 102 disposed on a surface of the substrate 110, a porous graphene layer 114 coated on the at least one electrode 102, and an adhesion layer 105 disposed between the at least one electrode 102 and the porous graphene layer 114 for enhancing the binding of the porous graphene layer 114 to the at least one electrode 102. Further, the present invention provides for a method 200 for preparing the electrode module.

## Description

The present invention relates to an electrode module for stimulation of the central and/or peripheral nervous system and/or recording of one or more signals of the central and/or peripheral nervous system.

Further, the present invention relates to a lead device for stimulation of the central and/or peripheral nervous system and/or recording of one or more signals of the central and/or peripheral nervous system, preferably for neurostimulation and recording.

Still further, the present invention relates to a method for preparing an electrode module for stimulation of the central and/or peripheral nervous system and/or recording of one or more signals of the central and/or peripheral nervous system.

In practice it is known that deep brain stimulation is a method for the diagnosis and therapy of neurodegenerative diseases such as inter alia the Parkinson's disease, epilepsy, and chronic pain. In this respect, electrical stimulation by means of leads which were implanted into brain regions like the subthalamic nucleus and/or the globus pallidus internus can for example alleviate the tremor symptoms of a patient suffering from a drug-resistant Parkinson's disease. Further, the signals from a brain region at which the leads were implanted can be recorded and the state of the brain tissue can be determined using impedance measurements.

Typically, such leads have an array of electrodes arranged thereon for interfacing with neural tissue, wherein the number and shape of the electrodes as well as the specific arrangement of the electrodes on the lead determines the electrical stimulation wave which affects the brain tissue.

Standard electrodes are usually made of platinum Pt, platinum-iridium (Pt/lr), iridium oxide (IrOx) or titanium nitride (TiN). Those materials interact with the living tissue through a combination of Faradaic and capacitive currents, but may offer a limited chemical stability and may be rigid. Metals' performance may strongly drop in microelectrodes of tens of micrometres in diameter. Further, metals can degrade over continuous tissue stimulation.

In order to avoid some of these disadvantages, electrodes with a coating applied to an electrode base material were suggested. In this regard graphene based (coating) materials are very promising. This is not only because these materials are highly inert and mechanically robust and therefore avoid degradation problems. Graphene based materials are highly favorable also because of their electrical properties and flexibility. Graphene based materials thus appear ideal for safe electrical interfacing in aqueous environments. However, such coatings may bring along other challenges which include mechanical stability of the coatings when the electrode is in operation/use.

In general, when in use, such electrodes or array of electrodes being manufactured as a thin-film substrate or the like is folded and/or wrapped, e.g., around a stylet of the lead, and/or moved and/or heavily used in another way. This can either weaken the thin-film substrate or it is possible that at least one layer is delaminated. The same problems also apply to an electrode or array of electrodes, wherein the electrodes are provided with a coating, such as described in, e.g., the European Patent Application Nr. 21382496.4.

However, long term stability, in particular mechanical stability, of such devices is also a crucial aspect to consider for implants, e.g., leads, in which such devices are integrated and/or embedded.

Therefore, it is an object of the present invention to advantageously develop an electrode module like the aforementioned kind, in particular an electrode module being more robust and/or durable, in particular with respect to delamination, and having improved performance.

This object is achieved by an electrode module for stimulation of the central and/or peripheral nervous system and/or recording of one or more signals of the central and/or peripheral nervous system comprising the features of claim 1. Advantageous embodiments of the present invention are particularly described in the dependent claims.

In this respect, an electrode module for stimulation of the central and/or peripheral nervous system and/or recording of one or more signals of the central and/or peripheral nervous system is provided, wherein the electrode module comprises:
- an electrode module per-product, in particular a stimulator lead, including a substrate and at least one electrode disposed on a surface of the substrate (110) or embedded in the substrate,
- a porous graphene layer coated on the at least one electrode, and
- an adhesion layer disposed between the at least one electrode and the porous graphene layer for enhancing the binding of the porous graphene layer to the at least one electrode (e.g. an adhesion layer underneath or the porous layer).

In particular, the electrode module comprises a top layer; and at least one electrode being disposed in or on the top layer, wherein the at least one electrode has at least one side facing away from the top layer, wherein the at least one side of the at least one electrode is coated with a layer of graphene oxide, and wherein the at least one side of the at least one electrode is partially covered by a portion of the top layer.

Particularly, the basic idea of the present invention is to provide a reliable electrode module having improved properties and a robust structure. In particular, the performance, structural integrity and durability of the electrode module is improved. The electrode module comprises a layer of Graphene oxide (GO) bonded by an adhesion layer onto the metallic electrodes. To ensure the longevity of the layer of reduced graphene oxide on the at least one electrode, measures are implemented to mitigate any potential delamination issues, so that the delamination within the electrode module or the layer of graphene oxide on the at least one electrode throughout its operational lifespan is prevented. In this respect, the electrode module comprises a top layer, partially covering the coted of reduced GO. In particular, a portion of the top layer may function as mechanical retaining means in view of the layer of graphene oxide and, especially, the layer of graphene oxide together with the at least one electrode.

In particular, the electrode module pre-product, e.g. a stimulator lead, comprises a paddle lead, a percutaneous lead, an intracortical lead, an intra vascular lead or an intra nerve lead.

In particular, the electrode module further comprises a top layer or a cover layer placed or disposed on the substrate and partially covering the porous graphene layer, e.g. a peripheral region of the porous graphene layer coated on the at least one electrode.

The at least one electrode may comprise an edge portion, wherein the edge portion may be at least partially covered by the portion of the top layer such that the at least one electrode may be partially sandwiched within and by the top layer. Since the edge portion, e.g., an area on the edge portion, of the at least one electrode may be at least partially covered by the portion of the top layer, it may be ensured that the area of the at least one electrode being exposed for engaging with tissue may be maximized, e.g. may have a maximum amount, while being reliably secured by the portion of the top layer.

In particular, the porous graphene layer comprises a layer of reduced, sputtered graphene oxide nanoparticle, a stack of reduced graphene flakes, a layer of carbon nanotubes or a layer of carbon nanopillars.

Alternatively or additionally, the at least one side of the at least one electrode together with the layer of reduced graphene oxide may be partially covered by the portion of the top layer. Hence, not only the at least one electrode is reliably secured by the portion of the top layer as described above, but the area of the layer of reduced graphene oxide being exposed for engaging with tissue may also be maximized, e.g. may also have a maximum amount, while being reliably secured by the portion of the top layer.

Alternatively or additionally, the at least one side of the at least one electrode may be covered by about 5% to about 20%, in particular about 5% to about 10%, by the portion of the top layer. In this respect, different mechanical loads may be withstood in dependence to the coverage of the at least one side of the at least one electrode by the portion of the top layer. In other words, the electrode module may provide an enhanced adaptability with respect to the mechanical load of its intended application.

The portion of the top layer may define a substantially continuous or a substantially discontinuous covering area on the at least one side of the at least one electrode. In this respect, a substantially continuous covering area may be obtained in a simple and/or cost-effective manner during production and/or protection against delamination may easily be ensured. However, a substantially discontinuous covering area may provide a maximum amount of area being exposed for engaging with tissue, in particular while protection against delamination may easily be ensured. Therefore, the electrode module may achieve an enhanced flexibility with respect to a desired goal depending on the intended application (e.g., cost efficiency, electrode engagement of tissue, etc.), while protection against delamination may be ensured.

In particular, the substrate comprises a planar thin-film substrate, a sheet or a cylindrical substrate. For example, the substrate comprises a plurality of layers.

In particular, the top layer comprises a polymer such as polyimide, parylene, polydimethylsiloxane, and SU8 (e.g. an epoxy-based photoresist).

In particular, the substrate, e.g. in the form of one layer or a plurality of layers, comprises a polymeric material such as polyurethane, silicon-polyurethane, polyurea, polyurethane-urea, polyethylene, fluoropolymers (e.g. PTFE), liquid crystal polymer or polyester.

The electrode module may further comprise at least one further layer disposed next to the top layer, wherein the at least one further layer may house at least one electrically conductive connection being assigned to the at least one electrode. This may provide a simple structure for electrically connecting the at least one electrode and for protecting the at least one electrically conductive connection from environmental effects.

The at least one electrode may extend from the top layer to the at least one further layer and therethrough such that the at least one electrode may further have a further side facing away from the at least one further layer, wherein the further side of the at least one electrode may be covered by a further layer of reduced graphene oxide, and wherein the further side of the at least one electrode may be partially covered by a portion of the at least one further layer. Therefore, the at least one electrode may engage tissue in at least two directions being opposite to each other for stimulation of the central and/or peripheral nervous system and/or recording of one or more signals of the central and/or peripheral nervous system in an enlarged manner with respect to area and/or volume, in particular while also having the before mentioned advantages.

The at least one further layer may comprise at least one further electrode being disposed in or on the at least one further layer, wherein the at least one further electrode may have at least one side facing away from the at least one further layer, wherein the at least one side of the at least one further electrode may be covered by a further layer of reduced graphene oxide, and wherein the at least one side of the at least one further electrode may be partially covered by a portion of the at least one further layer. Therefore, the at least one further electrode may engage tissue in a direction being opposite to the direction of the at least one electrode for stimulation of the central and/or peripheral nervous system and/or recording of one or more signals of the central and/or peripheral nervous system in an enlarged manner with respect to area and/or volume, in particular while also having the before mentioned advantages.

In particular, the substrate comprises at least one electrically conductive connection electrically connected to the at least one electrode.

The at least one further layer may comprise a bottom layer comprising said portion of the at least one further layer; and at least one intermediate layer disposed between the top layer and the bottom layer, wherein the at least one intermediate layer and/or the bottom layer may house the at least one electrically conductive connection being assigned to the at least one electrode. Therefore, this multi-layer structure of the electrode module may enable each electrode to be controlled via a single layer, i.e. that the corresponding electrically conductive connection arranged in a single layer of an electrode module may be assigned to a specific electrode. However, it may also be possible that more than one electrode may be controlled via a single layer.

It may be conceivable that at least one of the layers, e.g., the top layer, the at least one further layer, the at least one intermediate layer, and/or the bottom layer, may be made of a polymer, in particular a silicone, preferably a medical grade silicone. Therefore, the portion partially covering the at least one electrode may be formable in a simple manner, e.g., may be easily manufactured.

In particular, the at least one electrode comprises metal or metal alloys such as Au, Pt, IrOX or TiN, Ptlr. For example, the at least one electrode comprises a disc electrode, a ring electrode or an array of electrodes.

Additionally or alternatively, it may be conceivable that the at least one electrode and/or the at least one further electrode may be made of platinum Pt, platinum-iridium (Pt/lr), iridium oxide (IrOx), or titanium nitride (TiN). Those materials may reliably conduct a combination of Faradaic and capacitive currents for interacting with living tissue.

Additionally or alternatively, it may be conceivable that the at least one electrically conductive connection may be made of a superalloy, such as MP35N, or gold (Au), or copper(Cu).

The present invention further provides a lead device for stimulation of the central and/or peripheral nervous system and/or recording of one or more signals of the central and/or peripheral nervous system, preferably for neurostimulation and recording, wherein the lead device comprises at least one electrode module as described above and/or in the following.

It should be understood that the electrode module may additionally or alternatively be suited to be integrated and/or embedded in other kinds of lead devices being suitable for other applications than the before-mentioned application. In more general words, a lead device for medical use may be provided, which may comprise at least one electrode module as described above and/or in the following.

It should be further understood that any feature described in connection with the electrode module may also be part of the lead device, and vice versa. It should be further understood that any advantage and/or property described in connection with the electrode module may also apply to the lead device, and vice versa.

Additionally, it should be understood that the present invention further encompasses a/the use of an/the electrode module for stimulation of the central and/or peripheral nervous system and/or recording of one or more signals of the central and/or peripheral nervous system, and that the present invention further encompasses a/the use of a/the lead device for stimulation of the central and/or peripheral nervous system and/or recording of one or more signals of the central and/or peripheral nervous system, preferably for neurostimulation and recording, wherein the lead device comprises at least one electrode module as described above and/or in the following.

The present invention further provides a method for preparing an electrode module for stimulation of the central and/or peripheral nervous system and/or recording of one or more signals of the central and/or peripheral nervous system, in particular an electrode module as described above and/or in the following.

In particular, the method comprises the steps of
- providing an electrode module pre-product, e.g. a stimulator lead, which at least comprises a substrate and at least one electrode (102) disposed on a surface of the substrate; and
- coating a porous graphene layer on the at least one electrode (e.g. aloso one the substrate).

For example, the method comprises the steps of providing an electrode module pre-product which at least comprises a top layer, and at least one electrode being disposed in or on the top layer, wherein the at least one electrode has at least one side facing away from the top layer; coating the at least one side of the at least one electrode with reduced graphene oxide; and partially covering the at least one side of the at least one electrode with a portion of the top layer.

In particular, the method further comprises a first treatment process, e.g. for adding primers, such as chemical adhesion promotors on to the at least one electrode Pt, Ptlr in the form of foil or sheet.

For example, the first treating step is performed on the electrode module prior to coating the porous graphene layer on the at least one electrode. The first treatments step comprises adding or coating an adhesion layer on the at least one electrode.

In particular, the first treatment process comprises at least one of a chemical vapor deposition process, a self-assembled monolayer process and a spray coating process.

In particular, the method further comprises securing the coated porous graphene layer onto the substrate and/or the at least one electrode, preferably said securing comprises placing a top layer on the porous graphene layer and pattering or cutting the top layer to reveal (uncover or expose) the areas of the porous graphene layer.

For example, the securing of the coated porous graphene layer comprises depositing or coating a cover layer over or onto the layer of porous graphene and/or the substrate, and selectively removing sections of the cover layer to uncover or expose areas of the porous graphene layer deposited onto the at least one electrode. For example, the selective removal of the top layer maintains the cover layer intact on a peripheral region of the at least one electrode and/or central areas thereof.

For example, the step of pattering and/or cutting comprises techniques such as lithography, laser edging or laser ablation, plasma edging, RIE edging, high-resolution water edging, chemical edging.

Advantageously, the step of pattering or cutting facilitate a controlled and precise removal of the top layer, enabling intricate and fine-edged patterns

In particular, the step of coating or adding the graphene layer can be performed by way of Chemical Vapor Deposition, GO filtration and GO membrane transfer, spray coating process, polyimide spin coating, sputtering, evaporation or aqueous dispersion, or a laser induced graphene process. For example, the porous graphene layer comprises a reduced graphene oxide layer.

The step of partially covering may comprise partially over-molding and/or partially over-depositing the at least one side of the at least one electrode with the same material, of which the top layer is made of, to thereby, at the same time, integrally form the portion of the top layer itself. The step of partially covering may be obtained by thin film technology as known in the art, e.g., by chemical deposition, in particular chemical vapor deposition or atomic layer deposition, and/or physical deposition, in particular physical vapor deposition. Hence, the at least one side of the at least one electrode may be partially covered with a portion of the top layer in a simple and/or cost-effective manner.

Prior to coating, the at least one side of the at least one electrode may be surface treated by the first treatment process.

The step of coating may comprise transferring reduced graphene oxide from a sacrificial substrate to the at least one side of the at least one electrode or sputtering reduced graphene oxide on the at least one side of the at least one electrode.

Transferring and/or sputtering may comprise an application of pressure, in particular a pressure from 10⁵ to 4 x 10⁸ Pa, and/or temperature, in particular a temperature from 100 to 240 °C, for a predetermined time, in particular a time period from 1 min to 24 h.

Prior to transferring, the graphene oxide, e.g., a graphene oxide structure, may be formed on the sacrificial substrate.

Preparing of said graphene oxide / graphene oxide structure may comprise filtering a graphene oxide solution through a porous membrane thereby forming a graphene oxide structure on the membrane top.

In particular, the graphene oxide solution may be an aqueous solution, optionally, wherein the concentration of graphene oxide in the solution may be from 0.001 to 5 mg/mL, and the volume filtered may be from 5 to 1000 mL.

In particular, the graphene oxide solution may be provided comprising graphene flakes having COOH-, OH- and O-groups and the graphene flakes may be dispersed in water.

Preparing of said graphene oxide / graphene oxide structure may further comprise transferring the graphene oxide structure from the membrane onto said sacrificial substrate, whereby the graphene oxide structure is placed between the membrane at the top and the sacrificial substrate at the bottom.

Preparing of said graphene oxide / graphene oxide structure may further comprise removing the membrane, whereby the graphene oxide structure remains attached onto the sacrificial substrate.

In particular, the method further comprises a second treatment process, e.g. Iterative Intermediate treatments, preferably prior to the step of securing. The second treatment process comprises the step of creating a 3D structure with multiple layers, such as atomic layer deposition process (ALD), followed by the step of edging (plasma, laser or reactive ion edging).

The second treatment process may further comprise the step of repeating the steps of creating and edging to increase layer thickness, porosity.

The second treatment process employs a layer-by layer deposition of thin films with uniform thickness.

The ALD process offers advantages for creating functional coatings and intricate nanostructures or complex 3D structures in a precise manner.

In particular, the method further comprises a third treatment process for treating the coated graphene layer (post coating treatments).

For example, the third treatment step comprises chemical or thermal reduction, baking, thermal (or pressure) preforming, shaping or metal memory.

Advantageously, the third treatment process provides for an electrode module having improved electrical conductivity, tunable material properties, enhanced structural integrity and stability, shape memory effects, and improved mechanical properties, depending on the specific needs

It should be understood that any feature described in connection with the electrode module and/or the lead device may also be part of the method, and vice versa. It should be further understood that any advantage and/or property described in connection with the electrode module and/or the lead device may also apply to the method, and vice versa.

These and other aspects of the present invention will now be described in more detail, with reference to the appended drawings showing exemplary embodiments of the invention, wherein:
- **Fig. 1**: schematically illustrates a cross-sectional view of an electrode module according to the present invention;
- **Fig. 2**: schematically illustrates a state of preparing the electrode module of **Fig. 1****,** not indicating the first treatment step;
- **Fig. 3**: schematically illustrates a state of preparing the electrode module of **Fig. 1****,** indicating the first treatment step;
- **Fig. 4**: schematically illustrates a cross-sectional view of an electrode module according to a further exemplary embodiment of the present invention, and
- **Fig. 5**: schematically illustrates a method for preparing an electrode module.
Identical or functionally equivalent elements are denoted with the same reference signs throughout the figures.

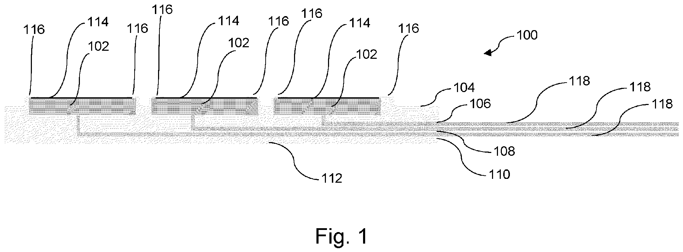

Turning to **Fig. 1****,** a cross-sectional view of an electrode module 100 according to an exemplary embodiment of the present invention is schematically illustrated.

The electrode module 100 is intended and/or configured for stimulation of the central and/or peripheral nervous system and/or recording of one or more signals of the central and/or peripheral nervous system.

The electrode module per-product 120, for example, a stimulator lead. The electrode module 100 comprises a substrate 110 and at least one electrode 102 disposed on a surface of the substrate 110 or embedded therein.

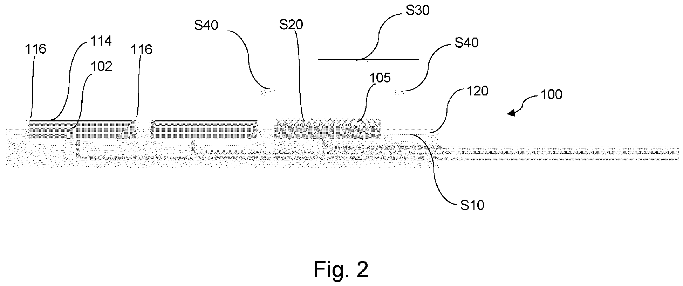

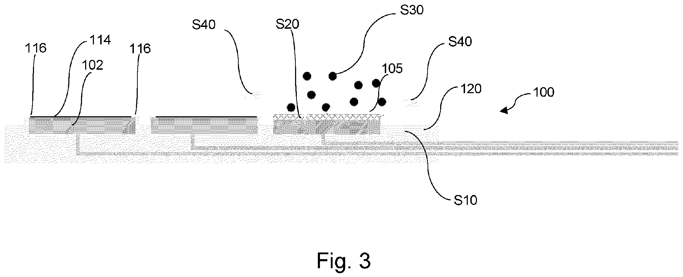

The electrode module further comprises a porous graphene layer 114 that is coated onto the at least one electrode 102, and an adhesion layer 105 (visible in Fig. 2 and 3) disposed between the at least one electrode 102 and the porous graphene layer 114. The adhesion layer helps to enhance binding of the porous graphene layer 114 to the at least one electrode 102.

Since conventional electrode materials such as metal, IrOx, or TiN exhibits inferior performance, the properties of the electrode module are improved by applying a layer of graphene (e.g. reduce graphene oxide or reduced graphene oxide in the form of flakes to the electrodes.

The electrode module 100, in this example, comprises three electrodes 102, a top layer 104, a first intermediate layer 106, a second intermediate layer 108, a third intermediate layer 110 and a bottom layer 112.

The electrodes 102 are disposed in the top layer 104 and comprise a side facing away from the top layer 104, which are exposed for engaging tissue, in particular for stimulation of the central and/or peripheral nervous system and/or recording of one or more signals of the central and/or peripheral nervous system.

Said side of each electrode 102 is coated with a layer of reduced graphene oxide 114.

An edge portion of each electrode 102 encompassing said respective side is at least partially covered by a portion 116 of the top layer 104.

Hence, the respective electrode 102 is partially sandwiched within and by the top layer 104.

In other words, said side of each electrode 102 is partially covered by the portion 116 of the top layer 104 such that each electrode 102 is partially sandwiched within and by the top layer 104.

In particular, said side of each electrode 102 together with the layer of reduced graphene oxide 114 is partially covered by the portion 116 of the top layer 104.

In this regard, said side of each electrode 102 is covered by about 5% to about 20%, in particular about 5% to about 10%, by the portion 116 of the top layer 104.

The portion 116 of the top layer 104 defines a substantially continuous covering area on each side of each electrode 102. In other words, the portion 116 protrudes onto said side of each electrode 102 over a whole corresponding side edge of each electrode 102 towards a respective center of each electrode 102, thereby defining said substantially continuous covering area.

Accordingly, delamination of the electrode module 100 can be reliably prevented and/or the layer of reduced graphene oxide 114 on each electrode 102 can be reliably secured thereon over its lifetime.

In this respect, the portion 116 of the top layer 104 functions as mechanical retaining means in view of the layer of reduced graphene oxide 114.

The top layer 104, the first intermediate layer 106, the second intermediate layer 108, the third intermediate layer 110 and the bottom layer 112 are stacked onto each other, wherein said layers 104, 106, 108, 110, 112 are depicted without borderlines in the **Fig. 1** to **Fig. 3** for reasons of clarity.

The top layer 104, the first intermediate layer 106, the second intermediate layer 108, the third intermediate layer 110 and the bottom layer 112 are made of a medical grade polymer.

Each intermediate layer 106, 108, 110 houses one electrically conductive connection 118 being respectively assigned to one electrode 102. However, it should be understood that one or more electrically conductive connections 118 may alternatively or additionally be housed in the top and/or bottom layer 104, 112. It should be further understood that more than one electrically conductive connection 118 may additionally or alternatively be housed in a, e.g., one, respective layer 104, 106, 108, 110, 112.

The electrically conductive connections 118 are made of gold.

Further, the electrodes 102 are made of platinum or titanium or any suitable medical grade materials.

Now turning to **Fig. 2** and **Fig. 3****,** a respective state of preparing the electrode module 100 of Fig. 1 are schematically illustrated.

In particular, **Fig. 2** and **Fig. 3** schematically illustrate a respective state of a method for preparing the electrode module 100. The different steps of the method are schematically depicted on one electrode 102 as an explanatory example.

The method comprises the step of providing (S10) an electrode module pre-product 120.

The electrode module pre-product at least comprises the three electrodes 102, the top layer 104, the first intermediate layer 106, the second intermediate layer 108, the third intermediate layer 110, the bottom layer 112 and the electrically conductive connections 118.The method optionally comprises the step of surface treating (S20) said side of each electrode 102.

The step of surface treatment (S20), i.e. the first treatment step, comprises the step of adding a layer of primers (e.g. chemical adhesion promotors), i.e. an adhesion layer 105, onto the surface of the substrate 110 and/or the at least one electrode 102.

For example, the first treatment step can be conducted using methods such as a chemical vapor deposition process, self-assembled monolayer process or spray coating process.

The method further comprises the step of coating (S30) said side of each electrode 102 with a layer of reduced graphene oxide 114.

As indicated in **Fig. 2****,** the step of coating (S30) comprises transferring the layer of graphene oxide 114 from a sacrificial substrate to said side of each electrode 102.

Alternatively, as indicated in **Fig. 3****,** the step of coating (S30) comprises sputtering reduced graphene oxide on said side of each electrode 102, to thereby form the layer of reduced graphene oxide 114.

It may be conceivable that transferring and/or sputtering further comprise an application of pressure, in particular a pressure from 10⁵ to 4 x 10⁸ Pa, and/or temperature, in particular a temperature from 100 to 240 °C, for a predetermined time, in particular a time period from 1 min to 24 h.

The method further comprises the step of partially covering (S40) said side of each electrode 102 with the portion 116 of the top layer 104, i.e. the step of securing the coated graphene layer.

The step of partially covering (S40) may comprise partially over-molding and/or partially over-depositing said side of each electrode 102 with the same material, of which the top layer 104 is made of, to thereby, at the same time, integrally form the portion 116 of the top layer 104 itself. It may be conceivable that the step of partially covering (S40) may be obtained by thin film technology as known in the art, e.g., by chemical deposition, in particular chemical vapor deposition or atomic layer deposition, and/or physical deposition, in particular physical vapor deposition.

It should be understood that any feature described in connection with the electrode module 100 can also be part of the method, and vice versa. It should be further understood that any advantage and/or property described in connection with the electrode module 100 can also apply to the method, and vice versa.

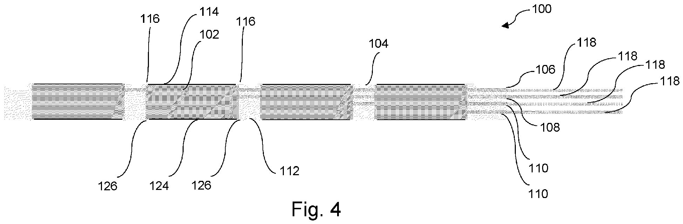

Now turning to **Fig. 4****,** a cross-sectional view of an electrode module 100 is schematically illustrated.

It should be understood that the electrode module 100 of **Fig. 4** is substantially the same as described above, in particular, with respect to the electrode module of Fig. 1, so that only the differences shall be described in the following.

As can be seen in **Fig. 4****,** the electrode module 100 additionally comprises a fourth electrode 102 and a fourth intermediate layer 122, which houses a further electrically conductive connection 118 being assigned to the fourth electrode 102.

The top layer 104, the first intermediate layer 106, the second intermediate layer 108, the third intermediate layer 110, the fourth intermediate layer 122 and the bottom layer 112 are stacked onto each other, wherein said layers 104, 106, 108, 110, 112, 122 are also depicted without borderlines in the **Fig. 4** for reasons of clarity.

As schematically shown in **Fig. 4****,** the electrodes 102 extend from the top layer to the bottom layer 112.

In other words, the electrodes 102 extend from the top layer to the bottom layer 112 through each intermediate layer 106, 108, 110, 122.

Each electrode 102 comprises a further side facing being opposite to the before-mentioned side of each electrode 102 and being oriented away from the bottom layer 112.

Said further side of each electrode 102 is covered by a further layer of reduced graphene oxide 124 in the same manner as described in connection with **Fig. 1** with respect to the side of each electrode 102.

Said further side of each electrode 102 is also partially covered by a portion 126 of the bottom layer 112 in the same manner as described in connection with **Fig. 1** with respect to the portion 116 of the top layer 104.

It may also be conceivable that one or more further electrodes may be disposed in or on the bottom layer 112, wherein each further electrode may have at least one side facing away from the bottom layer 112, wherein the at least one side of each further electrode may be covered by said further layer of reduced graphene oxide 124, and wherein the at least one side of the each further electrode may be partially covered by said portion 126 of the bottom layer 112.

It should be understood that the electrode module 100 of **Fig. 4** may be prepared in the same manner as described in connection with **Fig. 1** to **Fig. 3****,** wherein said further side may additionally be coated with reduced graphene oxide and then partially covered by the portion 126 of the bottom layer 112.

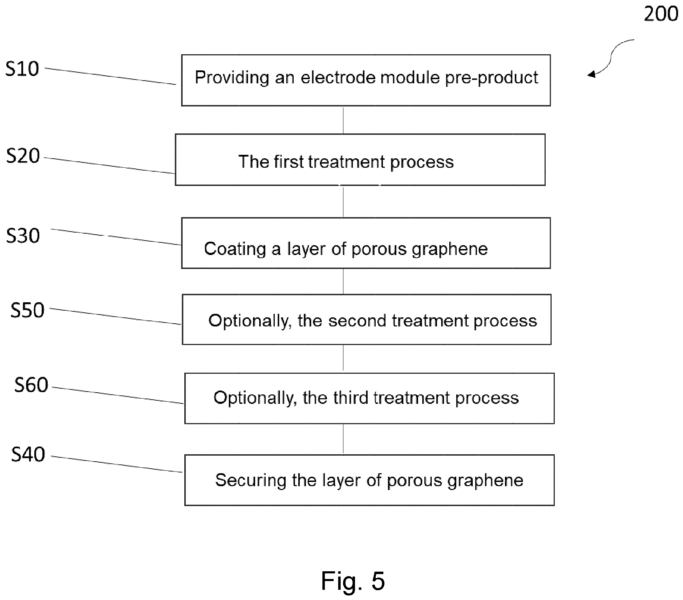

Fig. 5 shows a method 200 for preparing an electrode module 100 for stimulation of the central and/or peripheral nervous system and/or recording of one or more signals of the central and/or peripheral nervous system.

The method 200 comprises the following steps:
- providing (S10) an electrode module pre-product 120, in particular a stimulator lead, comprising a substrate 110 and at least one electrode 102;
- a first treatment process (S20) for treating the surface of the electrode module pre-product (120), in particular the surface of the at least one electrode 102;
- coating (S30) a porous graphene layer 114 onto the at least one electrode 102;
- optionally, a second treatment process (S50) for creating a 3D structures on the coated graphene layer (114),
- optionally, a third treatment process (S60) for treating the coated graphene layer 114, preferably the third treatment step comprises chemical or thermal reduction, baking, or thermal or pressure preforming.
- securing (S40) the coated graphene layer 114 onto the substrate 110 and/or the at least one electrode 102, preferably said securing S40 comprises placing a top layer 104 on the porous graphene layer 114 followed by pattering (or cutting) the top layer 104 to reveal the areas of the porous graphene layer 114.

### Reference Numerals

- 100: electrode module
- 102: electrode
- 104: top layer
- 105: adhesion layer
- 106: first intermediate layer
- 108: second intermediate layer
- 110: third intermediate layer
- 112: bottom layer
- 114: layer of reduced graphene oxide
- 116: portion of top layer
- 118: electrically conductive connection
- 120: electrode module pre-product
- 122: fourth intermediate layer
- 124: further layer of reduced graphene oxide
- 126: portion of bottom layer

- S10: step
- S20: step
- S30: step
- S40: step
- S50: step
- S60: step

## Claims

1. An electrode module (100) for stimulation of the central and/or peripheral nervous system and/or recording of one or more signals of the central and/or peripheral nervous system, comprising:
- an electrode module per-product (120), in particular a stimulator lead, including a substrate (110) and at least one electrode (102) disposed on a surface of the substrate (110),
- a porous graphene layer (114) coated on the at least one electrode (102), and
- an adhesion layer (105) disposed between the at least one electrode (102) and the porous graphene layer (114) for enhancing the binding of the porous graphene layer (114) to the at least one electrode (102).

2. The electrode module (100) according to claim 1,
**characterized in that**
the electrode module (100) further comprises a top layer (104) disposed on the substrate (110) and partly covers a peripheral region of the porous graphene layer (114) on the at least one electrode (102).

3. The electrode module (100) according to claim 1 or 2,
**characterized in that**
the porous graphene layer (114) comprises a layer of reduced, sputtered graphene oxide nanoparticles (114), a stack of reduced graphene flakes, a layer of carbon nanotubes or a layer of carbon nanopillars.

4. The electrode module (100) according to any one of claims 1 to 3,
**characterized in that**
the adhesion layer (105) comprises a self-assembled monolayer including a molecular chain with a head group for binding to the at least one electrode and a functional group for binding to the porous graphene layer, preferably the molecular chain comprises at least eight consecutive covalently bonded molecules or atoms.

5. The electrode module (100) according to any one of the preceding claims,
**characterized in that**
the substrate (110) comprises a planar thin-film substrate, a sheet or a cylindrical substrate.

6. The electrode module (100) according to any one of the proceedings claims,
**characterized in that**
the electrode module per-product (120) comprises a paddle lead, a percutaneous lead, an intracortical lead, an intra vascular lead or an intra nerve lead.

7. The electrode module (100) according to any one of the preceding claims,
**characterized in that**
the top layer (104) comprises a polymer such as polyimide, perylene, polydimethylsiloxane, and an epoxy-based photoresist.

8. The electrode module (100) according to any one of the preceding claims,
**characterized in that**
the substrate (110) comprises a polymeric material such as polyurethane, silicon-polyurethane, polyurea, polyurethane-urea, polyethylene.

9. The electrode module (100) according to any one of the preceding claims,
**characterized in that**
the at least one electrode (102) comprises metal or metal alloys such as Au, Pt, IrOx or TiN, Ptlr, preferably the at least one electrode (102) comprising a disc electrode, a ring electrode or an array of electrodes.

10. The electrode module (100) according to any of the proceedings claims,
**characterized in that**
the substrate (110) comprises at least one electrically conductive connection (118) electrically connected to the at least one electrode (102), preferably the at least one electrically conductive connection comprising a superalloy, such as MP35N, Au, or Cu.

11. A lead device for stimulation of the central and/or peripheral nervous system and/or recording of one or more signals of the central and/or peripheral nervous system, preferably for neurostimulation and recording, wherein the lead device comprises at least one electrode module (100) according to one of claims 1 to 10.

12. A method for preparing an electrode module (100) for stimulation of the central and/or peripheral nervous system and/or recording of one or more signals of the central and/or peripheral nervous system, in particular an electrode module (100) according to one of claims 1 to 10, wherein the method comprises the following steps:
- providing (S10) an electrode module pre-product (120), in particular a stimulator lead, which at least comprises a substrate (110) and at least one electrode (102) disposed onto a surface of the substrate (110); and
- coating (S30) a porous graphene layer (114) onto the at least one electrode (102).

13. The method (200) according to claim 12,
**characterized in that**
the method (200) further comprises a first treatment process (S20) for treating the electrode module pre-product (120) prior to coating the layer of porous graphene onto the at least electrode (102), preferably the first treatment step comprising coating an adhesion layer (105) onto the at least one electrode (102).

14. The method (200) according to claim 12 or 13,
**characterized in that**
the method further comprises a second treatment process (S50) for creating a 3D structures on the coated graphene layer (114), and/or
a third treatment process (S60) for treating the coated graphene layer (114), preferably the third treatment step comprising chemical or thermal reduction, baking, or thermal or pressure preforming.

15. The method (200) according to any one of claims 12 to 14,
**characterized in that**
the method further comprises securing (S40) the coated graphene layer (114) onto the substrate (110) and/or the at least one electrode (102), preferably said securing (S40) comprising placing a top layer (104) on the porous graphene layer (114) and pattering the top layer (104) to reveal the areas of the porous graphene layer (114).
